(19) Europäisches Patentamt
European Patent Office
Office européen des brevets

(11) Publication number: **0 404 797 B1**

(12) # EUROPEAN PATENT SPECIFICATION

(45) Date of publication of patent specification: **01.02.95**

(51) Int. Cl.$^{6}$: **C07D 471/04**, A61K 31/435, //(C07D471/04,235:00,221:00)

(21) Application number: **89903310.4**

(22) Date of filing: **15.03.89**

(86) International application number:
**PCT/US89/00975**

(87) International publication number:
**WO 89/08653 (21.09.89 89/23)**

(54) **1H/3H-[4-(N,N-CYCLOALKYL AND/OR BRANCHED-ALKYLCARBOXAMIDO)-BENZYL]IMIDAZO[4,5-c]PYRIDINES AS PAF ANTAGONISTS.**

(30) Priority: **15.03.88 US 168491**

(43) Date of publication of application:
**02.01.91 Bulletin 91/01**

(45) Publication of the grant of the patent:
**01.02.95 Bulletin 95/05**

(84) Designated Contracting States:
**AT BE CH DE FR GB IT LI LU NL SE**

(56) References cited:
**EP-A- 142 801**
**US-A- 4 568 680**

**CHEMICAL ABSTRACTS, Vol. 109, No. 9, 29 August 1988, Columbus, Ouio, USA; Manley et al.: "Preparation and testing of imidazopyridines as platelet-activating factor (PAF) antagonists", abstract no. 73438c.**

(73) Proprietor: **G.D. Searle & Co.**
**P.O. Box 5110**
**Chicago**
**Illinois 60680 (US)**

(72) Inventor: **WEIER, Richard, M.**
**240 Hickory Court**
**Lake Bluff, IL 60044 (US)**
Inventor: **KHANNA, Ish, K.**
**8922 Bronx Avenue**
**Skokie, IL 60077 (US)**

(74) Representative: **DELETED Beil, Walter (deceased 22.07.83)**
**BEIL, WOLFF & BEIL**
**Rechtsanwälte**
**Adelonstrasse 58**
**D-6230 Frankfurt am Main 80 (DE)**

EP 0 404 797 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

RELATED APPLICATION

This application is a continuation-in-part of U.S. Application Ser. No. 07/168,491 filed March 15, 1988.

FIELD OF THE INVENTION

This invention is in the field of mammalian therapeutics and relates to compounds for treatment of mammalian diseases such as inflammation, cardiovascular disorders, asthma and other diseases. Of particular interest is a class of novel N-dicycloalkyl or N-branched-alkyl benzamides of certain imidazopyridine derivatives useful for treatment of cardiovascular and immuno-inflammatory related disorders mediated by platelet activating factor (PAF).

BACKGROUND OF THE INVENTION

Chronic platelet dysfunction or hyperactivity of platelets is associated with many chronic diseases such as cerebrovascular disease, ischemic heart disease, diabetic retinopathy, angina, peripheral vascular disease and myocardial infarction.

Platelet activation results in platelet aggregrate formation and the release of substances which can accelerate platelet aggregation. Platelet activation may be stimulated by any of several aggregatory agents through various mechanisms depending upon the particular disorder at hand. Aggregatory agents, such as collagen, thromboxane $A_2$ ($TXA_2$), adenosine diphosphate (ADP), adrenaline, 5-hydroxytryptamine, thrombin, or platelet activating factor (PAF), may act directly on receptors resulting in platelet aggregation, or may alter cyclic-AMP levels or calcium ion sequestration to produce platelet aggregation.

Platelet-activating factor (PAF) has been associated with various biological activities and pathways, thus making it an important mediator responsible for a variety of physiological processes, including activation of platelets, smooth muscle contraction, pathogenesis of immune complex deposition, inflammation, and respiratory, cardiovascular and intravascular alterations. These physiological processes are associated with a large group of diseases, such as cardiovascular disorders, asthma, lung edema, endotoxin shock, adult respiratory distress syndrome and inflammatory diseases.

Various classes of compounds are known for inhibiting platelet activation induced by agents such as arachidonic acid, collagen and platelet activating factor. For example, several classes of imidazole derivatives are known for use in treatment of various cardiovascular and immuno-response diseases related to platelet dysfunction or hyperactivity. U.S. Patent No. 2,025,946 to Iizuki et al mentions certain classes of imidazoles, namely, N-($\omega$-substituted alkylphenylalkyl)imidazoles, N-($\omega$-substituted alkylphenyl)-imidazoles and N-(nucleus-substituted phenylalkyl)-imidazoles which are described as having inhibitory effect on thromboxane synthetase and to be useful for treatment of inflammation, thrombus and asthma. U.S. Patents No. 4,284,641 and No. 4,416,895 to Thorogood describe certain cycloalkyl/cycloalkenyl imidazoles which inhibit platelet aggregation or reduce the adhesive character of platelets by selective inhibition of thromboxane $A_2$. Also described for the same purpose in U.S. Patent No. 4,537,340 to Thorogood is a class of 1-arylalkylimidazoles. In U.S. Patent No. 4,243,671 to Harris et al, the compound 1-(3-phenyl-2-propenyl)1H-imidazole is described as effective in inhibiting thromboxane synthetase, arachidonic acid-induced platelet aggregation and bronchoconstriction.

Compounds are known for use in treating platelet dysfunction or hyperactivity induced specifically by platelet activating factor (PAF). For example, a certain class of glycerol derivatives useful as PAF antagonists is described in European Application No. 142,333. A class of indene derivatives is described in European Application No. 142,801 as PAF inhibitors. Compounds containing heterocyclic moieties of various types are also known as PAF antagonists. U.S. Patent No. 4,579,862 to Manley et al describes certain imidazole/pyridinyl-alkanoic acid derivatives as PAF antagonists. South African Application No. 87/6817 (corresponding to EP-A-260 613) describes a class of imidazopyridine derivatives useful as PAF inhibitors and mentions, in particular, the compound N-cyclohexyl-N-methyl-4-(1H-imidazo[4,5-c]pyridin-1-yl-methyl)-benzamide as an inhibitor of PAF-induced aggregation in an assay using human platelet-rich plasma.

DESCRIPTION OF THE INVENTION

Treatment of platelet-related pathologies, such as PAF-stimulated pathologies or platelet-mediated airway hyper-reactivity, in a mammal is accomplished by administering to a susceptible mammal a therapeutically-effective amount of a compound of the class represented by Formula I:

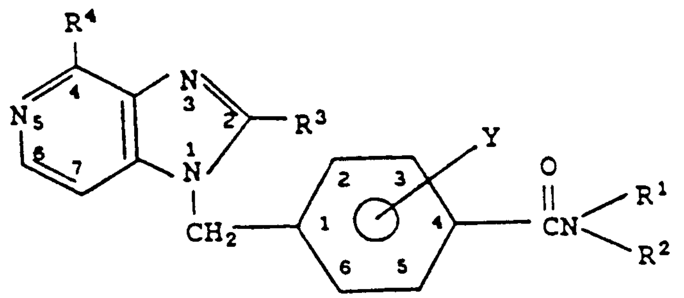

(I)

wherein Y is hydrido at all substitutable positions or Y is one or two groups attached at positions selected from the two, three, five and six positions of the phenyl ring with Y independently selected from fluoro, methyl and methoxy; wherein each of $R^1$ and $R^2$ is independently selected from isopropyl, sec-butyl, cyclopentyl, methylcyclopentyl, cyclohexyl and methylcyclohexyl; and wherein each of $R^3$ and $R^4$ is hydrido.

Compounds of most interest of Formula I are those wherein the substitutable positions on the nitrogen atom of the benzamide moiety are substituted as follows: (a) substitution with cycloalkyl groups such as cyclopentyl, cyclohexyl, methylcyclopentyl and methylcyclohexyl; or (b) substitution with branched alkyl groups which have configurations which mimic the presence of the aforementioned cycloalkyl groups, such branched alkyl groups being isopropyl, sec-butyl; or (c) substitution with groups from (a) and (b), above.

Examples of compounds of Formula I having the benzamide nitrogen substituted with two cycloalkyl groups, or two branched alkyl groups, or a cycloalkyl and a branched alkyl group, are those of presented in Table I, below:

## Table I - Preferred Compounds

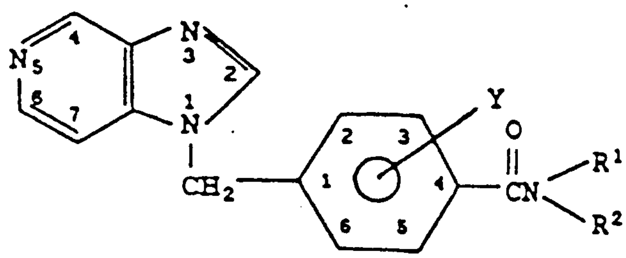

1H-{4-[N-($R^1$)-N-($R^2$)-carboxamido]-Y-benzyl}imidazo[4,5-c]pyridine

| Compound # | Substitution | | |
|---|---|---|---|
| | $R^1$ | $R^2$ | Y |
| 1 | cyclopentyl | cyclopentyl | - |
| 2 | cyclopentyl | cyclohexyl | - |
| 3 | cyclopentyl | 3-methylcyclohexyl | - |
| 4 | 2-propyl | cyclohexyl | 2-methoxy |
| 5 | 2-propyl | cyclohexyl | - |
| 7 | cyclopentyl | cyclopentyl | 2-fluoro |
| 8 | cyclohexyl | cyclohexyl | - |
| 9 | sec-butyl | cyclopentyl | - |
| 10 | sec-butyl | cyclohexyl | - |
| 15 | sec-butyl | cyclopentyl | 2-methoxy |
| 16 | sec-butyl | cyclohexyl | 2-methoxy |

Of the foregoing specific examples, most preferred are the following:
1H-[4-(N,N-dicyclopentylcarboxamido)benzyl]imidazo[4,5-c]pyridine;
1H-[4-(N-cyclopentyl-N-cyclohexylcarboxamido)benzyl]imidazo[4,5-c]pyridine;
1H-[4-(N-cyclopentyl-N-3-methylcyclohexylcarboxamido)benzyl]imidazo[4,5-c]pyridine;
1H-[4-(N-2-propyl-N-cyclohexylcarboxamido)-2-methoxybenzyl]imidazo[4,5-c]pyridine;
1H-[4-(N-2-propyl-N-cyclohexylcarboxamido)benzyl]-[4,5-c]pyridine; and.

Included within the classes and sub-classes of compounds embraced by Formula I are the tautomeric forms of the described compounds, isomeric forms including diastereoisomers and enantiomers, and the pharmaceutically-acceptable salts thereof. The term "pharmaceutically-acceptable salts" embraces salts commonly used to form alkali metal salts and to form addition salts of free acids or free bases. Since the compounds of Formula I contain basic nitrogen atoms, such salts are typically acid addition salts or quaternary salts. The nature of the salt is not critical, provided that it is pharmaceutially acceptable, and acids which may be employed to form such salts are, of course, well known to those skilled in this art. Examples of acids which may be employed to form pharmaceutically acceptably acid addition salts include such inorganic acids as hydrochloric acid, sulphuric acid and phosphoric acid, and such organic acids as maleic acid, succinic acid and citric acid. Other pharmaceutically acceptably salts include salts with alkali metals or alkaline earth metals, such as sodium, potassium, calcium and magnesium, or with organic bases, such as dicyclohexylamine. All of these salts may be prepared by conventional means by reacting, for example, the appropriate acid or base with the corresponding compound of Formula I.

Synthetic Procedures

Compounds of Formula I may be prepared in accordance with the following general procedures: An imidazo[4,5-c]pyridine intermediate, as shown in its tautomeric mixture form (IV)

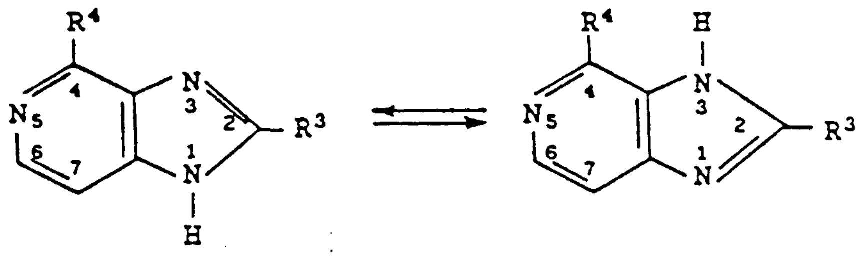

(IV)

is reacted with an alkylbenzamide intermediate (V)

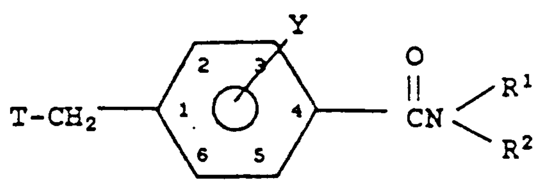

(V)

wherein each of Y and $R^1$ through $R^5$ is selected as described above, and T is selected from chloro, bromo, iodo, an alkylsulfonyloxy such as methanesulfonyloxy and an arylsulfonyloxy such as para-toluenesulfonyloxy, with T preferably being bromo. The alkylation reaction of the imidazo[4,5-c]pyridine intermediate (IV) with a suitable alkylbenzamide intermediate (V) can be carried out in accordance with the following general procedure:
Imidazopyridine (10 mmole) is dissolved in a dipolar aprotic solvent such as DMF and stirred at 0-25 °C. To this a base such as sodium hydride (10-15 mmole), potassium hydride (10-15 mmole), potassium-t-butoxide (10-15 mmole), or a complex of potassium-t-butoxide (10-15 mmole) with 18-crown-6 (1 mmole), is added over 10 minutes. The reaction mixture is stirred at room temperature for 1-6 hours. The reaction flask is cooled (0-20 °C) and a solution of substituted benzyl bromide in a dipolar aprotic solvent is added over 15-20 minutes. The reaction mixture is stirred for 4-16 hours. After quenching with acid (1 N HCl or acetic acid), part of the solvent is removed under reduced pressure. The reaction is diluted with ethyl acetate and the organic layer is washed with saturated $K_2CO_3$, water and brine. After drying over $Na_2SO_4$, the solvent is removed and the residue is chromatographed (silica gel, $CH_2Cl_2$: methanol: $NH_4OH$ 90:10:1) to give the desired product.

Preparation of Imidazo[4,5-c]pyridine (IV)

An example of a specific method for preparation of the imidazo[4,5-c]pyridine intermediate, from which the general preparation of intermediate (IV), above, may be derived, is as follows: A stirred mixture of 3,4-diaminopyridine (10.0 g, 0.092 mol) and formic acid (20 ml) was heated under reflux for 2.5 hours. The resulting mixture was cooled and the formic acid was evaporated off under reduced pressure to yield a residue. The residue was dissolved in ethanol (30 ml) at 80 °C and the resulting solution was treated with calcium carbonate (10 g) and neutralized by stirring under reflux for 1 hour. The hot mixture was filtered and the residue was washed with hot ethanol (3 x 300 ml). The filtrate and washings were combined and the ethanol was evaporated off under reduced pressure to yield 3,4-diformylaminopyridine as a colorless solid. The 3,4-diformylaminopyridine was heated at 200-220 °C and 66.5 Pa (0.5 mm Hg) pressure in a Kugelrohr bulb-to-bulb distillation apparatus to yield a crude product as a distillate which solidified on cooling. The crude product was recrystallized from ethyl acetate to yield imidazo[4,5-c]pyridine as a colorless crystalline solid, having a melting point of 174-176 °C and the following physical characteristics: 1H-NMR (δ, DMSO-$d^6$): 6.60 (broad s,1H), 7.66 (dd,1H), 8.37 (d,1H), 8.44 (s,1H) and 9.00 (d,1H).

Preparation of Alkylbenzamide Intermediates (V)

The alkylbenzamide intermediate (V), suitably substituted on the amide nitrogen with two cycloalkyl groups, or two branched alkyl groups, or both a cycloalkyl group and a branched alkyl group, may be prepared by procedures generally known in the art [see, for example, V.S. Chauhan et al, Int.J. Peptide Protein Res., 15, 96-101 (1980)]. Preparation of suitable secondary amine precursors to the alkylated benzamide intermediate (V) is generally known [see, for example, W.S. Emerson, Org. Reactions, 4, 174 (1948); and J.B. Campbell et al, "Catalysis in Organic Synthesis", p. 43, Academic Press, New York (1980)-]. Key alkylbenzamide intermediates (V) may be prepared in accordance with the following generalized procedures:

General Method for Preparation of Secondary Amines:

To a solution of ketone (0.1 mol), amine (0.1 mol), and acetic acid in ethanol (100 mL) in a Parr bottle was added 5% Pd/C (1 g). The bottle was sealed, purged with nitrogen and pressurized 414 x $10^3$pa (60 psi) with hydrogen. The reaction was stirred at room temperature (2-20 hours) until the theoretical amount of hydrogen was taken up. The reaction vessel was vented, purged with nitrogen and filtered. The clear filtrate was concentrated, the residue dissolved in ethyl acetate and washed with saturated aqueous potassium carbonate, water and brine. After drying over $MgSO_4$, the solvent was removed to give the crude product which was distilled under pressure to provide product having sufficient purity for the amidation reaction.

General Method of Preparation of 4-Methyl-3-Methoxy and 3-Fluoro Benzamides and Amides of α-Bromo-p-Toluic Acid

The acid chlorides were prepared from the corresponding carboxylic acids by refluxing in thionyl chloride (2 molar excess) for two hours. Excess thionyl chloride was removed by azeotrope four times with toluene (150 ml portions). The residual acid chloride was dissolved in THF (12 ml/g of acid) and cooled to about -10 °C. A solution of two molar equivalents of the amine in THF (10 ml/g of amine) was added dropwise with stirring. When addition was completed, the reaction was allowed to warm to room temperature and stirred for 1-2 hours. The reaction was quenched with 1N HCl, diluted with $H_2O$, and extracted three times with ethyl acetate (150 ml portions). The combined organic layers were washed three times with saturated aqueous sodium bicarbonate solution (200 ml portions), once with water (300 ml), three times with saturated aqueous sodium chloride solution (200 ml portions) and dried over sodium sulfate). The drying agent was filtered and the filtrate concentrated under reduced pressure to give a crude product that was chromatographed on silica gel using mixtures of ethyl acetate and hexane to give the purified amide.

General Method for the Preparation of: 4-Bromomethyl-3-Methoxy and -3-Fluoro Benzamides

A stirred mixture of the amide and N-bromosuccinimide (NBS) (1:1 molar ratio) in carbon tetrachloride was irradiated with a sun lamp for 1-3 hours. A white precipitate was filtered and washed with a minimum amount of $CHCl_3$. The filtrate was washed with water and the aqueous layer, after basification with ammonium hydroxide, was extracted three times with chloroform (150 ml portions). All organic layers were combined, washed three times with saturated aqueous sodium chloride solution and dried over sodium sulfate. The drying agent was filtered and the filtrate concentrated under reduced pressue to give a crude product that was chromatographed on silica gel using mixtures of ethyl acetate and hexane to give the purified bromomethyl compound.

The following Examples 1-6 are detailed descriptions of the key synthetic step for preparing compounds of the invention. These detailed preparations fall within the scope of, and serve to exemplify, the more generally described procedures above. These Examples 1-6 are presented for illustrative purposes only. Most of the commercially-available materials were obtained from Aldrich Chemical Co., Milwaukee, Wis.

Example 1

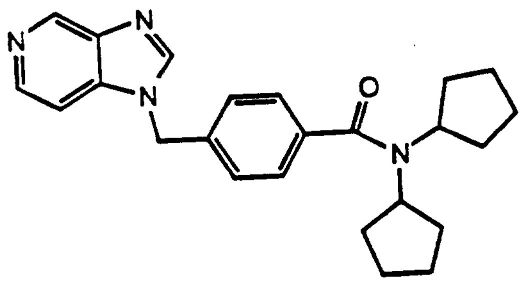

1a

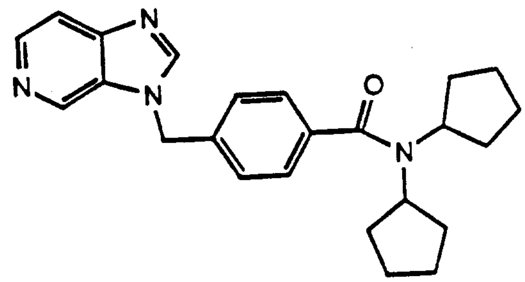

1b

1a: 1H-[4-(N,N-dicyclopentylcarboxamido)benzyl]imidazo-[4,5-c]pyridine

1b: 3H-[4-(N,N-dicyclopentylcarboxamido)benzyl]imidazo-[4,5-c]pyridine

To a solution of imidazopyridine (2.5 g, 21 mmol) in DMF (100 ml) at 0 °C, sodium hydride (1.3 g, 60% dispersion in mineral oil, 32.5 mmol) was added over 15 min. in 4 portions. The reaction mixture was stirred at 0 °C for 1 hour and then at room temperature for 2 hours. After recooling (0-5 °C), a solution of N,N-dicyclopentyl-4-bromomethyl benzamide (7.35 g, 21 mmol) in DMF (50 ml) was added over 5-10 min. The reaction was allowed to warm to room temperature and stirred for 4 hours. After carefully quenching with 1N HCl, some DMF was removed under reduced pressure. The crude residue was diluted with ethyl acetate and the organic layer was washed sequentially with saturated potassium carbonate, water and brine. The organic layer was concentrated to give 7.9 g of the residue. After chromatography (silica gel, $CH_2Cl_2/MeOH/NH_4OH$ 90/10/1), Products 1a (700 mg) and 1b (442 mg) were isolated.

Both 1a and 1b were crystallized from EtOAc/$CH_3CN$ (85/15) to give white crystalline compounds.

| | Anal. | Calc. | Found | M.P. °C |
|---|---|---|---|---|
| Cpd. 1a: $C_{24}H_{28}N_4O \cdot 0.2H_2O$ | C<br>H<br>N | 73.54<br>7.32<br>14.30 | 73.57<br>7.45<br>14.31 | 227-228 |
| Cpd. 1b: $C_{24}H_{28}N_4O$ | C<br>H<br>N | 74.22<br>7.21<br>14.43 | 74.11<br>7.0<br>14.48 | 212-213 |

Example 2

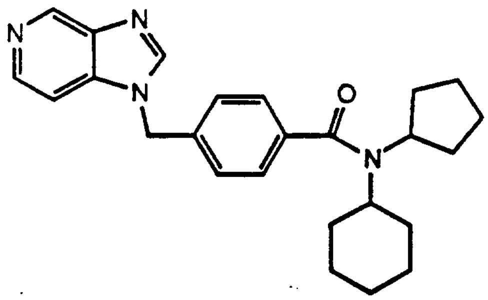

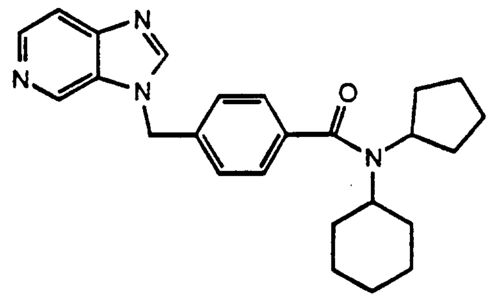

2a 2b

2a: 1H-[4-(N-cyclopentyl-N-cyclohexylcarboxamido)benzyl]imidazo[4,5-c]pyridine

2b: 3H-[4-(N-cyclopentyl-N-cyclohexylcarboxamido)benzyl]imidazo[4,5-c]pyridine

To a solution of imidazopyridine (1.5 g 12.59 mmol) in DMF (50 mL), sodium hydride (755 mg, 60% dispersion in mineral oil, 18.9 mmol) was added over 15 min. at 0-5°C. After stirring at room temperature for 8 hours, a solution of N-cyclopentyl-N-cyclohexyl-4-bromomethylbenzamide (5.5 g, 15.11 mmol) in 50 mL DMF was added over 10 min. The reaction was stirred at room temperature for about 18 hours and quenched with 1N HCl. Part of the solvent was removed under reduced pressure and the reaction contents were diluted with ethyl acetate. The organic layer was washed with saturated potassium carbonate, water and brine. After drying over $MgSO_4$, the solvent was removed and the residue chromatographed (silica gel; $CH_2Cl_2$: EtOH:$NH_4OH$ 75:25:1) to give 2a (443 mg, 9%) and 2b (133 mg, 3%).

| | Anal. | Calc. | Found | M.P. °C |
|---|---|---|---|---|
| Cpd. 2a: $C_{25}H_{30}N_4O \cdot 0.5H_2O$ | C<br>H<br>N | 72.96<br>7.59<br>13.61 | 72.80<br>7.46<br>13.50 | 228-229 |
| Cpd. 2b: $C_{25}H_{30}N_4O \cdot 0.25H_2O$ | C<br>H<br>N | 73.26<br>7.56<br>13.76 | 73.96<br>7.54<br>13.75 | 222-224 |

Example 3

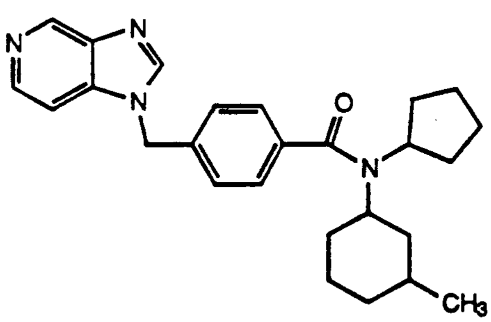

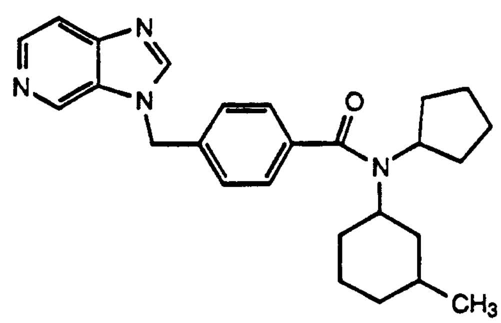

3a 3b

3a: 1H-[4-(N-cyclopentyl-N-3-methylcyclohexylcarboxamido)benzyl]imidazo[4,5-c]pyridine

3b: 3H-[4-(N-cyclopentyl-N-3-methylcyclohexylcarboxamido)benzyl]imidazo[4,5-c]pyridine

To a solution of imidazopyridine (1.19 g, 10 mmol) in DMF (50 mL), sodium hydride (660 mg, 60% dispersion in mineral oil, 15 mmol) is added over 15 min. at 0-5 °C. After stirring at room temperature for 5 hours, a solution of N-cyclopentyl-N-3-methylcyclo-hexyl-4-bromomethylbenzamide (3.78 g, 10 mmol) is added over 15 min. The reaction is stirred for about 8 hours and quenched with 1N HCl. Part of the solvent is removed under reduced pressure and the reaction contents are diluted with ethyl acetate. The organic layer is washed with saturated potassium carbonate, water and brine. After drying over $MgSO_4$, the solvent is removed and the residue chromatographed (silica gel; $CH_2Cl_2$:MeOH:$NH_4OH$ 90:10:1) to give pure product.

Example 4

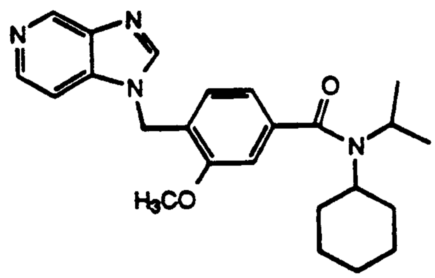

4a

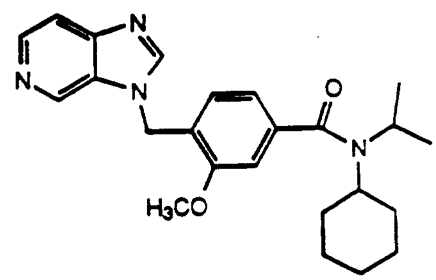

4b

4a: 1H-[4-(N-2-propyl-N-cyclohexylcarboxamido)-2-methoxybenzyl]imidazo[4,5-c]pyridine

4b: 3H-[4-(N-2-propyl-N-cyclohexylcarboxamido)-2-methoxybenzyl]imidazo[4,5-c]pyridine

To a solution of imidazopyridine (1.2 g, 10.08 mmol) in DMF (50 mL), sodium hydrido (605 mg, 60% dispersion in mineral oil, 15.2 mmol) was added over 15 min. at 0-5 °C. After stirring at room temperature for 8 hours, a solution of 3-methoxy-4-bromomethyl-N-isopropyl,N-cyclohexylbenzamide (4.5 g, 12.1 mmol) in 50 mL DMF was added over 10 min. The reaction was stirred at room temperature for about 18 hours and quenched with 1N NCl. Part of the solvent was removed under reduced pressure and the reaction contents were diluted with ethyl acetate. The organic layer was washed with saturated potassium carbonate, water and brine. After drying over $MgSO_4$, the solvent was removed and the residue (5.4 g) chromatographed (silica gel; $CH_2Cl_2$:$CH_3OH$: $NH_4OH$ 85:15:1) to give 4a (993 mg, 24%) and 4b (666 mg, 16%).

| | Anal. | Calc. | Found | M.P. °C |
|---|---|---|---|---|
| Cpd. 4a: $C_{24}H_{30}N_4O_2$ | C<br>H<br>N | 70.90<br>7.43<br>13.78 | 70.80<br>7.54<br>13.67 | 160-161 |
| Cpd. 4b: $C_{24}H_{30}N_4O_2$ | C<br>H<br>N | 73.26<br>7.56<br>13.76 | 73.96<br>7.54<br>13.75 | 222-224 |

Example 5

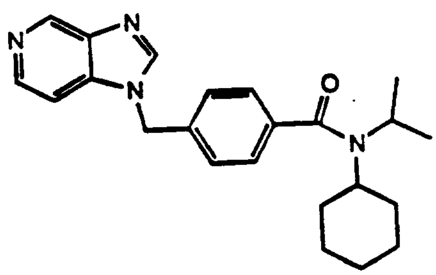

5a

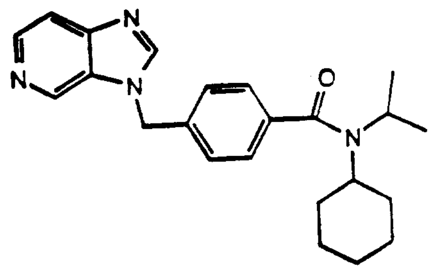

5b

5a: 1H-[4-(N-2-propyl-N-cyclohexylcarboxamido)benzyl]imidazo[4,5-c]pyridine

5b: 3H-[4-(N-2-propyl-N-cyclohexylcarboxamido)benzyl]imidazo[4,5-c]pyridine

To a solution of imidazopyridine (1.4 g, 11.8 mmol) in DMF (50 mL), sodium hydride (710 mg, 60% dispersion in mineral oil, 17.7 mmol) was added over 15 min. at 0-5 °C. After stirring at room temperature for 8 hours, a solution of N-isopropyl-N-cyclohexyl-4-bromomethylbenzamide (4.8 g, 14.2 mmol) in 50 mL DMF was added over 10 min. The reaction was stirred at room temperature for about 18 hours and quenched with 1N HCl. Part of the solvent was removed under reduced pressure and the reaction contents were diluted with ethyl acetate. The organic layer was washed with saturated potassium carbonate, water and brine. After drying over $MgSO_4$, the solvent was removed and the residue (4.1 g) chromatographed (silica gel; $CH_2Cl_2$ : $CH_3OH$ : $NH_4OH$ 90:10:1) to give 5a (1200 mg, 28%) and 5b (780 mg, 18%).

| | Anal. | Calc. | Found | M.P. °C |
|---|---|---|---|---|
| Cpd. 5a: $C_{23}H_{28}N_4O \bullet 0.25H_2O$ | C | 72.50 | 72.34 | 202-203 |
| | H | 7.25 | 7.48 | |
| | N | 14.71 | 14.58 | |
| Cpd. 5b: $C_{23}H_{28}N_4O$ | C | 73.37 | 73.34 | 233-234 |
| | H | 7.49 | 7.55 | |
| | N | 14.88 | 14.64 | |

This invention also relates to a method of treatment for patients (or mammalian animals raised in the dairy, meat, or fur industries or as pets) suffering from disorders or diseases which can be attributed to PAF as previously described, and more specifically, to a method of treatment involving the administration of a compound of Formula I as the active ingredient.

Accordingly, compound of the invention can be used among other things to reduce inflammation, to correct respiratory, cardiovascular, and intravascular alterations or disorders, and to regulate the activation or coagulation of platelets, the pathogenesis of immune complex deposition and smooth muscle contractions.

For the treatment of inflammation, cardiovascular disorder, asthma, or other diseases mediated by PAF, compound of the invention may be administered orally, topically, parenterally, or by inhalation spray or rectally in dosage unit formulations containing conventional non-toxic pharmaceutically acceptable carriers, adjuvants and vehicles. The term parenteral as used herein includes subcutaneous injections, intravenous, intramuscular, intrasternal injection or infusion techniques.

The compounds of the present invention may be administered by any suitable route, preferably in the form of a pharmaceutical composition adapted to such a route, and in a dose effective for the treatment intended. Therapeutically effective doses of the compounds of the present invention required to prevent or arrest the progress of the medical condition are readily ascertained by one of ordinary skill in the art.

Accordingly, the invention provides a class of novel pharmaceutical compositions comprising one or more compounds of the present invention in association with one or more non-toxic, pharmaceutically acceptable carriers and/or diluents and/or adjuvants (collectively referred to herein as "carrier" materials) and if desired other active ingredients. The compounds and composition may, for example, be administered intravascularly, intraperitoneally, subcutaneously, intramuscularly or topically.

For oral administration, the pharmaceutical composition may be in the form of, for example, a tablet, capsule, suspension or liquid. The pharmaceutical composition is preferably made in the form of a dosage unit containing a particular amount of the active ingredient. Examples of such dosage units are tablets or capsules. These may with advantage contain an amount of active ingredient from about 1 to 250 mg, preferably from about 25 to 150 mg. A suitable daily dose for a mammal may vary widely depending on the condition of the patient and other factors. However, a dose of from about 0.1 to 3000 mg/kg body weight, particularly from about 1 to 100 mg/kg body weight, may be appropriate.

The active ingredient may also be administered by injection as a composition wherein, for example, saline, dextrose or water may be used as a suitable carrier. A suitable daily dose is from about 0.1 to 100 mg/kg body weight injected per day in multiple doses depending on the disease being treated. A preferred daily dose would be from about 1 to 30 mg/kg body weight.

The dosage regimen for treating an infectious disease condition with the compounds and/or compositions of this invention is selected in accordance with a variety of factors, including the type, age, weight, sex and medical condition of the patient, the severity of the infection, the route of administration, and the particular compound employed, and thus may vary widely.

For therapeutic purposes, the compounds of this invention are ordinarily combined with one or more adjuvants appropriate to the indicated route of administration. If per os, the compounds may be admixed with lactose, sucrose, starch powder, cellulose esters of alkanoic acids, cellulose alkyl esters, talc, stearic acid, magnesium stearate, magnesium oxide, sodium and calcium salts of phosphoric and sulphuric acids, gelatin, acacia, sodium alginate, polyvinylpyrrolidone, and/or polyvinyl alcohol, and then tableted or encapsulated for convenient administration. Alternatively, the compounds may be dissolved in water, polyethylene glycol, propylene glycol, ethanol, corn oil, cottonseed oil, peanut oil, sesame oil, benzyl alcohol, sodium chloride, and/or various buffers. Other adjuvants and modes of administration are well and widely known in the pharmaceutical art. Appropriate dosages, in any given instance, of course depend upon the nature and severity of the condition treated, the route of administration, and the species of mammal involved, including its size and any individual idiosyncrasies.

Representative carriers, diluents and adjuvants include for example, water, lactose, gelatin, starches, magnesium stearate, talc, vegetable oils, gums, polyalkylene glycols, petroleum jelly, etc. The pharmaceutical compositions may be made up in a solid form such as granules, powders or suppositories or in a liquid form such as solutions, suspensions or emulsions. The pharmaceutical compositions may be subjected to conventional pharmaceutical operations such as sterilization and/or may contain conventional pharmaceutical adjuvants such as preservatives, stabilizers, wetting agents, emulsifiers, buffers, etc.

Dosage levels on the order from about 1 mg to about 100 mg per kilogram of body weight per day are useful in the treatment of the above-indicated conditions (from about 50 mg to about 5 mgs. per patient per day). For example, inflammation is effectively treated and anti-pyretic and analgesic activity manifested by the administration from about 25 to about 75 mg of the compound per kilogram of body weight per day (about 75 mg to about 3.75 gm per patient per day). Preferably, from about 5 mg to about 50 mg per kilogram of body weight per daily dosage should produce highly effective results (about 250 mg to about 2.5 gm per patient per day).

The amount of active ingredient that may be combined with the carrier materials to produce a - single dosage form will vary depending upon the host treated and the particular mode of administration. For example, a formulation intended for oral administration to humans may contain from 5 mg to 95 mg of active agent compounded with an appropriate and convenient amount of carrier material which may vary from about 5 to 95 percent of the total composition. Dosage unit forms will generally contain between from about 25 mg to about 500 mg of active ingredient.

It will be understood, however, that the specific dose level for any particular patient will depend upon a variety of factors including the activity of the specific compound employed, the age, body weight, general health, sex, diet, time of administration, route of administration, rate of excretion, drug combination and the severity of the particular disease undergoing therapy.

## Biological Evaluation

Compounds prepared in Examples 1 2, 4 and 5, were evaluated in an assay to determine their potency as selective antagonists of platelet activating factor (1-0-alkyl-2-acetyl-sn-glycero-3-phosphorylcholine or "AGEPC") which induces aggregation and secretion of [$^3$H] serotonin from labeled, washed rabbit platelets, by the following procedure:

Chemicals: All chemicals and solvents used were reagent grade or better, purchased from Sigma Chemical Co. (St. Louis, MO). Buffers for washing platelets were prepared fresh daily in double-glass-distilled water,

as previously described by C.P. Cox et al, Peptides, 5, 25-28 (1984). AGEPC was purchased from Bachem, Inc. (Torrance, CA) and dissolved in phosphate-buffered saline (PBS) containing 2.5 mg/ml bovine serum albumin (BSA).

AGEPC Antagonists: Stock solutions (10 mM) of each of the antagonist compounds were prepared in dimethyl/sulfoxide and diluted in PBS prior to use.

Platelet Aggregation and Secretion: Washed, [$^3$H]serotonin-labeled rabbit platelets were prepared as described by C.P. Cox et al, Peptides, 5, 25-28 (1984), and maintained in an atmosphere of 5% $CO_2$ at 37°C until used in the bioassay. Aliquots of platelets (2.5 x $10^8$/ml) were incubated with either an antagonist of AGEPC or the appropriate vehicle for 60 seconds prior to the addition of AGEPC (0.2 nM to 0.2 $\mu$M). Aggregation was continuously monitored on a strip-chart recorder and recorded as the height of the tracing at 60 seconds after the addition of AGEPC. Secretion of [$^3$H]serotonin was measured in a sample of the platelet suspension removed at 60 seconds after the addition of AGEPC. The percent inhibition of aggregation and secretion was calculated by comparing antagonist-treated platelets with the appropriate vehicle-treated control platelets. Each combination of antagonist and AGEPC was repeated 12-15 times, using several different platelet preparations. $IC_{50}$ values were determined by inspection of the dose-response curves, and are reported below:

| Compound | Secretion ($IC_{50}$,M) | Aggregation ($IC_{50}$, M) |
|---|---|---|
| 1a | 14.6 X $10^{-9}$ | $10^{-6}$ to $10^{-7}$ |
| 1b | $10^{-6}$ to $10^{-7}$ | $10^{-5}$ to $10^{-6}$ |
| 2a | 9.1 X $10^{-9}$ | $10^{-7}$ to $10^{-8}$ |
| 2b | $10^{-6}$ to $10^{-7}$ | $10^{-5}$ to $10^{-6}$ |
| 4a | 4.8 X $10^{-9}$ | $10^{-7}$ to $10^{-8}$ |
| 4b | $10^{-6}$ to $10^{-7}$ | $10^{-5}$ to $10^{-6}$ |
| 5a | 8.3 X $10^{-9}$ | $10^{-7}$ to $10^{-8}$ |
| 5b | $10^{-5}$ to $10^{-6}$ | $10^{-5}$ to $10^{-6}$ |

## Claims

**1.** A compound of the formula

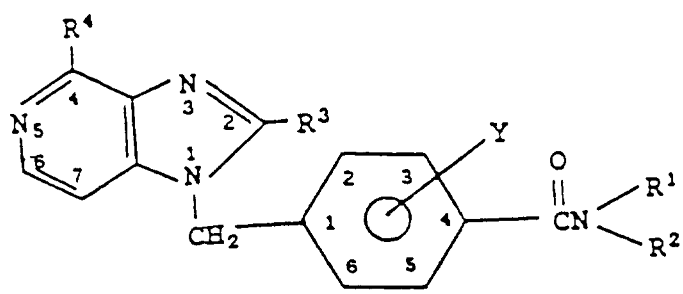

wherein Y is
hydrido at all substitutable positions or Y is one or two groups attached at positions selected from the two, three, five and six positions of the phenyl ring with Y independently selected from fluoro, methyl and methoxy; wherein each of $R^1$ and $R^2$ is independently selected from isopropyl, sec-butyl, cyclopentyl, methylcyclopentyl, cyclohexyl and methylcyclohexyl; and wherein each of $R^3$ and $R^4$ is hydrido.

**2.** Compound of Claim 1 selected from the group consisting of
1H-[4-(N,N-dicyclopentylcarboxamido)benzyl]imidazo[4,5-c]pyridine;
1H-[4-(N-cyclopentyl-N-cyclohexylcarboxamido)benzyl]imidazo[4,5-c]pyridine;
1H-[4-(N-cyclopentyl-N-3-methylcyclohexylcarboxamido)benzyl]imidazo[4,5-c]pyridine;
1H-[4-(N-2-propyl-N-cyclohexylcarboxamido)-2-methoxybenzyl]imidazo[4,5-c]pyridine; and
1H-[4-(N-2-propyl-N-cyclohexylcarboxamido)benzyl]-[4,5-c]pyridine.

3. A pharmaceutical composition comprising a therapeutically effective amount of a compound and a pharmaceutically-acceptable carrier or diluent, said compound selected from a family of compounds of the formula

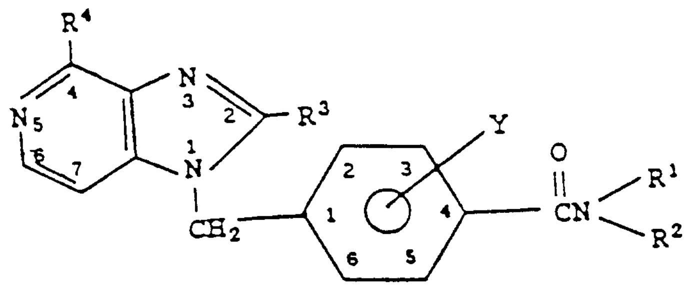

wherein Y is hydrido at all substitutable positions or Y is one or two groups attached at positions selected from the two, three, five and six positions of the phenyl ring with Y independently selected from fluoro, methyl and methoxy; wherein each of $R^1$ and $R^2$ is independently selected from isopropyl, sec-butyl, cyclopentyl, methylcyclopentyl, cyclohexyl and methylcyclohexyl; and wherein each of $R^3$ and $R^4$ is hydrido.

4. The composition of Claim 3 wherein said compound is selected from the group consisting of
1H-[4-(N,N-dicyclopentylcarboxamido)benzyl]imidazo[4,5-c]pyridine;
1H-[4-(N-cyclopentyl-N-cyclohexylcarboxamido)benzyl]imidazo[4,5-c]pyridine;
1H-[4-(N-cyclopentyl-N-3-methylcyclohexylcarboxamido)benzyl]imidazo[4,5-c]pyridine;
1H-[4-(N-2-propyl-N-cyclohexylcarboxamido)-2-methoxybenzyl]imidazo[4,5-c]pyridine;
1H-[4-(N-2-propyl-N-cyclohexylcarboxamido)benzyl]-[4,5-c]pyridine.

5. Use of one of the compounds of claims 1 to 2 for the preparation of a medicament against diseases attributable to platelet-related pathologies, such as PAF-stimulated pathologies, or platelet-mediated airway hyper-activity, in a mammal.

6. Use of one of the compounds of claims 1 to 2 for the preparation of a medicament against cardiovascular disorders, asthma, lung edema, endotoxin shock, adult respiratory distress, or an inflammatory disease.

## Patentansprüche

1. Eine Verbindung der Formel

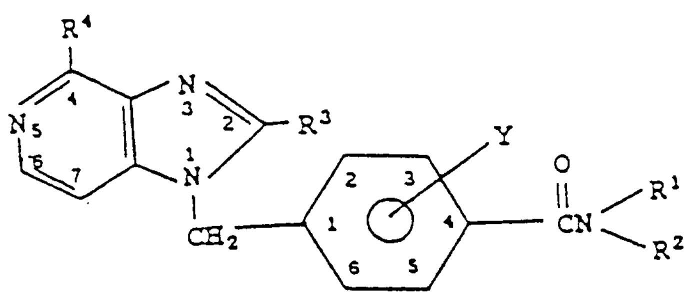

worin Y Hydrido an allen substituierbaren Positionen ist oder Y eine oder zwei Gruppen bedeutet, die an die Positionen gebunden sind, ausgewählt aus den 2-, 3-, 5- und 6-Positionen des Phenylringes, wobei Y unabhängig voneinander ausgewählt ist aus Fluor, Methyl und Methoxy; worin jeweils $R^1$ und $R^2$ unabhängig voneinander ausgewählt sind aus Isopropyl, sec-Butyl, Cyclopentyl, Methylcyclopentyl,

Cyclohexyl und Methylcyclohexyl und worin jeweils $R^3$ und $R^4$ Hydrido sind.

2. Verbindung nach Anspruch 1 ausgewählt aus der Gruppe bestehend aus
1H-[4-(N,N-Dicyclopentylcarboxamido)benzyl]imidazo-[4,5-c]pyridin;
1H-[4-(N-Cyclopentyl-N-cyclohexylcarboxamido)benzyl]imidazo[4,5-c]pyridin;
1H-[4-(N-Cyclopentyl-N-3-methylcyclohexylcarboxamido)-benzyl]imidazo-[4,5-c]pyridin;
1H-[4-(N-2-Propyl-N-cyclohexylcarboxamido)-2-methoxy-benzyl]imidazo-[4,5-c]pyridin und
1H-[4-(N-2-Propyl-N-cyclohexylcarboxamido)benzyl]-[4,5-c]-pyridin.

3. Eine pharmazeutische Zusammensetzung, enthaltend eine therapeutisch wirksame Menge einer Verbindung und einen pharmazeutisch verträglichen Träger oder ein Verdünnungsmittel, wobei diese Verbindung ausgewählt ist aus einer Familie von Verbindungen der Formel

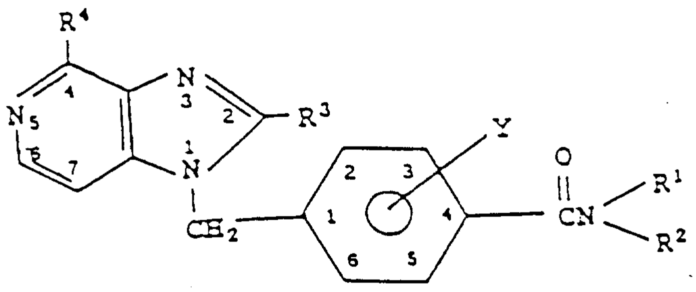

worin Y Hydrido an allen substituierbaren Positionen ist oder Y eine oder zwei Gruppen bedeutet, die an die Positionen gebunden sind, ausgewählt aus den 2-, 3-, 5- und 6-Positionen des Phenylringes, wobei Y unabhängig voneinander ausgewählt ist aus Fluor, Methyl und Methoxy; worin jeweils $R^1$ und $R^2$ unabhängig voneinander ausgewählt sind aus Isopropyl, sec-Butyl, Cyclopentyl, Methylcyclopentvl, Cyclohexyl und Methylcyclohexyl und worin jeweils $R^3$ und $R^4$ Hydrido sind.

4. Die Zusammensetzung nach Anspruch 3, worin diese Verbindung ausgewählt ist aus der Gruppe bestehend aus
1H-[4-(N,N-Dicyclopentylcarboxamido)benzyl]imidazo-[4,5-c]pyridin;
1H-[4-(N-Cyclopentyl-N-cyclohexylcarboxamido)benzyl]imidazo-[4,5-c]pyridin;
1H-[4-(N-Cyclopentyl-N-3-methylcyclohexylcarboxamido)-benzyl]imidazo-[4,5-c]pyridin;
1H-[4-(N-2-Propyl-N-cyclohexylcarboxamido)-2-methoxy-benzyl]imidazo-[4,5-c]pyridin und
1H-[4-(N-2-Propyl-N-cyclohexylcarboxamido)benzyl]-[4,5-c]-pyridin.

5. Verwendung einer der Verbindungen der Ansprüche 1 bis 2 zur Herstellung eines Medikamentes gegen Erkrankungen, die blutplättchen-abhängigen Pathologien zuzuschreiben sind, wie PAF-stimulierte Pathologien, oder blutplättchen-vermittelte Lutwegshyperaktivität in Säugern.

6. Verwendung einer der Verbindungen der Ansprüche 1 bis 2 zur Herstellung eines Medikamentes gegen cardiovasculäre Störungen, Asthma, Lungenödem, Endotoxin-Schock, Atemnot bei Erwachsenen oder eine entzündliche Erkrankung.

## Revendications

1. Composé de formule

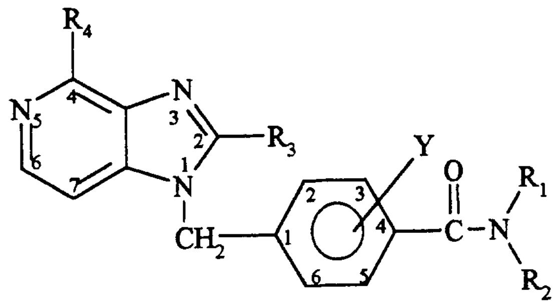

dans laquelle

Y représente un reste hydruro à toutes les positions pouvant être substituées, ou Y représente un ou deux groupes liés à des positions choisies parmi les positions 2, 3, 5 et 6 du noyau phényle, les Y étant choisis indépendamment parmi les restes fluoro, méthyle et méthoxy;

$R_1$ et $R_2$ sont choisis chacun indépendamment parmi les restes isopropyle, sec-butyle, cyclopentyle, méthylcyclopentyle, cyclohexyle et méthylcyclohexyle; et

$R_3$ et $R_4$ sont chacun un reste hydruro.

2. Composé selon la revendication 1, choisi dans le groupe constitué par
la 1H-[4-(N,N-dicyclopentylcarboxamido)benzyl]imidazo[4,5-c]pyridine;
la 1H-[4-(N-cyclopentyl-N-cyclohexylcarboxamido)benzyl]imidazo[4,5-c]pyridine;
la 1H-[4-(N-cyclopentyl-N-3-méthylcyclohexylcarboxamido)benzyl]imidazo[4,5-c]pyridine;
la 1H-[4-(N-2-propyl-N-cyclohexylcarboxamido)-2-méthoxybenzyl]imidazo[4,5-c]pyridine;
la 1H-[4-(N-2-propyl-N-cyclohexylcarboxamido)benzyl]imidazo[4,5-c]pyridine.

3. Composition pharmaceutique comprenant une quantité thérapeutiquement efficace d'un composé et un support ou diluant pharmaceutiquement acceptable, ledit composé étant choisi dans une famille de composés de formule

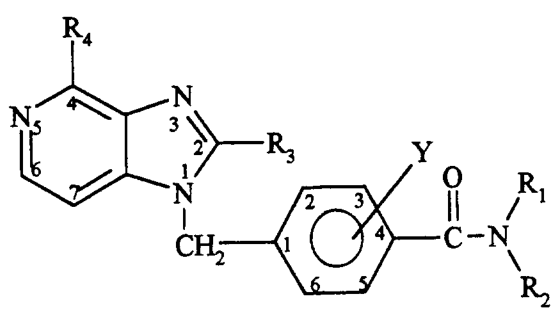

dans laquelle

Y représente un reste hydruro à toutes les positions pouvant être substituées, ou Y représente un ou deux groupes liés à des positions choisies parmi les positions 2, 3, 5 et 6 du noyau phényle, les Y étant choisis indépendamment parmi les restes fluoro, méthyle et méthoxy;

$R_1$ et $R_2$ sont choisis chacun indépendamment parmi les restes isopropyle, sec-butyle, cyclopentyle, méthylcyclopentyle, cyclohexyle et méthylcyclohexyle; et

$R_3$ et $R_4$ sont chacun un reste hydruro.

4. Composition selon la revendication 4, dans laquelle ledit composé est choisi dans le groupe constitué par
la 1H-[4-(N,N-dicyclopentylcarboxamido)benzyl]imidazo[4,5-c]pyridine;
la 1H-[4-(N-cyclopentyl-N-cyclohexylcarboxamido)benzyl]imidazo[4,5-c]pyridine;
la 1H-[4-(N-cyclopentyl-N-3-méthylcyclohexylcarboxamido)benzyl]imidazo[4,5-c]pyridine;
la 1H-[4-(N-2-propyl-N-cyclohexylcarboxamido)-2-méthoxybenzyl]imidazo[4,5-c]pyridine;
la 1H-[4-(N-2-propyl-N-cyclohexylcarboxamido)benzyl]imidazo[4,5-c]pyridine.

5. Utilisation de l'un des composés des revendications 1 à 2 pour la préparation d'un médicament contre les maladies imputables à des pathologies en rapport avec les plaquettes, comme les pathologies stimulées par le PAF, ou l'hyperactivité des voies aériennes à médiation plaquettaire, chez les mammifères.

6. Utilisation de l'un des composés des revendications 1 à 2 pour la préparation d'un médicament contre les troubles cardio-vasculaires, l'asthme, l'oedème pulmonaire, le choc endotoxinique, la détresse respiratoire de l'adulte ou des maladies inflammatoires.